# EUROPÄISCHE PATENTSCHRIFT

(11) **EP 1 171 175 B1**
(45) Veröffentlichungstag und Bekanntmachung des Hinweises auf die Patenterteilung: **04.10.2006**
(21) Anmeldenummer: 00934921.8
(22) Anmeldetag: 20.04.2000
(51) Int. Cl.: A61L 27/26, A61L 27/34, A61L 27/22, A61L 27/48

(54) **WIRKSTOFF-BESCHICHTETE ENDOPROTHESE MIT LANGZEITSTABILISIERUNG**
ACTIVE AGENT COATED ENDOPROSTHESIS WITH LONG-TERM STABILITY
ENDOPROTHESE REVETUE D' AGENT ACTIV A STABILISATION DE LONGUE DUREE

(30) Priorität: 20.04.1999 DE 19917696
(43) Veröffentlichungstag der Anmeldung: 16.01.2002
(73) Patentinhaber: SCHMIDT, Karlheinz, Prof. Dr. Dr., 72810 Gomaringen (DE)
(72) Erfinder: SCHMIDT, Karlheinz, Prof. Dr. Dr., 72810 Gomaringen (DE)
(74) Vertreter: Wüstefeld, Regine Marie
(86) Internationale Anmeldenummer: PCT/DE2000/001279
(87) Internationale Veröffentlichungsnummer: WO 2000/062834

(56) Entgegenhaltungen:
- WO-A-91/06324
- WO-A-93/20857
- US-A- 5 456 717
- US-A- 5 824 651
- ANSELME K: "Osteoblast adhesion on biomaterials" BIOMATERIALS,GB,ELSEVIER SCIENCE PUBLISHERS BV., BARKING, Bd. 21, Nr. 7, April 2000 (2000-04), Seiten 667-681, XP004188881 ISSN: 0142-9612

## Beschreibung

Die Erfindung betrifft die Verwendung eines Wirkstoffkomplexes für die Herstellung von biologischen Teilen, insbesondere von Organen für Lebewesen, mit den folgenden, voneinander unterschiedlichen und auf das jeweils herzustellende biologische Teil spezifisch abgestimmten Komponenten in Form mindestens einer Strukturkomponente auf der Basis von auf die Zellen des jeweils herzustellenden biologischen Teils spezifisch abgestimmtem extrazellulärem Material, mindestens einer Rekrutierungskomponente, mindestens einer Adhäsionskomponente und mindestens einer Wachstums- und/oder Maturationskomponente.

Im Stand der Technik ist bereits ein Wirkstoffkomplex für die Herstellung von biologischen Teilen, insbesondere von Organen für Lebewesen, mit den genannten Komponenten bekannt. Bei diesem bekannten Wirkstoffkomplex kann die Strukturkomponente beispielsweise aus verschiedenen Collagenen, Elastin oder Proteoglycanen bestehen. Als Rekrutierungskomponente für diesen Wirkstoffkomplex sind insbesondere Chemotaktica zu nennen, beispielsweise Peptide, wie N-F-Met-Leu-Phe- und/oder beispielsweise Metabolite der Arachidonsäure, wie Leukotriene. Die Aufgabe der Adhäsionskomponente können Eiweißkörper vom Typ des Fibronectins oder Laminins, aber auch Zell-Adhäsions-Moleküle, wie L-CAM, N-CAM und Matrix-Adhäsions-Moleküle, wie Cytotactin, Tenascin, Collagen Typ IV, V, VII, synthetische Peptide und Transmembran-Verbindungsproteine, wie Integrin, erfüllen. Die zunächst genannten Beispiele für Adhäsionskomponenten, Fibronectin und Laminin, sind für die Zwecke des hier erläuterten Wirkstoffkomplexes in den Bereich der Matrix-Adhäsions-Moleküle einzuordnen. Als weitere Komponente weist der genannte Wirkstoffkomplex mindestens eine Wachstums- und/oder Maturationskomponente auf, vorzugsweise in Form eines oder mehrerer Cytokine. Beispiele für solche Cytokine sind bei der Herstellung von Blut die Kolonie-stimulierenden Faktoren, bei der Herstellung von Bindegewebe der Fibroblasten Wachstumsfaktor, bei der Herstellung von Haut der epidermale Wachstumsfaktor, bei der Herstellung von Knorpel der Knorpel induzierende Faktor, bei der Herstellung der Milz oder der Lymphknoten der Lymphocyten aktivierende Faktor sowie Milzpeptide, für die Herstellung von Thymus der T-Zellen Wachstumsfaktor sowie Thymuspeptide, für die Herstellung von Knochen der Knochen-Wachstums-faktor sowie der transformierende Wachstumsfaktor, für die Herstellung von Blutgefäßen der Angiogenese-Faktor. Ferner finden noch die folgenden Cytokine Verwendung: Interleukine, Insulin-ähnliche Wachstumsfaktoren, der Tumor-Nekrose-Faktor, Prostaglandine, Leukotriene, transformierende Wachstumsfaktoren, von Thrombocyten abstammender Wachstumsfaktor, Interferone sowie von Endothelzellen abstammender Wachstumsfaktor.

Näheres über diesen Wirkstoffkomplex ist aus dem Europäischen Patent Nr. 0 500 556 zu entnehmen, dessen Offenbarungsgehalt hier ausdrücklich einbezogen wird.

Der vorliegenden Erfindung lag die Aufgabe zugrunde, den Wirkstoffkomplex einer breiteren Anwendung zugänglich zu machen.

Gelöst wird diese Aufgabe durch eine Verwendung des genannten Wirkstoffkomplexes zur Herstellung eines Endoprothesen-Implantats gemäß Anspruch 1. Hierdurch wird gegenüber den herkömmlich verwendeten, den Wirkstoffkomplex nicht aufweisenden Endoprothesen eine Langzeitstabilisierung der Endoprothese bewirkt.
Gemäß dieser erfindungsgemäßen Verwendung gelangt ein Wirkstoffkomplex gemäß Anspruch 1 zum Einsatz, der für die Herstellung von biologischen Teilen in Form von Knochen geeignet ist, und der die folgenden, voneinander unterschiedlichen und auf die Herstellung von Knochen spezifisch abgestimmten Komponenten in Form mindestens einer Strukturkomponente auf der Basis von auf die Zellen des herzustellenden Knochens spezifisch abgestimmtem extrazellulärem Material, mindestens einer Rekrutierungskomponente, mindestens einer Adhäsionskomponente und mindestens einer Wachstums- und/oder Maturationskomponente aufweist.

Dem Auffinden dieser erfindungsgemäßen Verwendung gingen umfangreiche Untersuchungen voraus, die die Kombination des Wirkstoffkomplexes mit verschiedenen, insbesondere mit metallischen Trägermaterialien zum Gegenstand hatten. Die Kombination eines Trägermaterials mit dem Wirkstoffkomplex ist nicht unproblematisch. Nach den bisher zur Verfügung stehenden Erkenntnissen über den Wirkstoffkomplex und seine komplexe Wirkungsweise ist zumindest eine Beeinträchtigung der Bildung oder Wiederherstellung des jeweiligen zu behandelnden biologischen Teils, z. B. der knöchernen Regeneration, zu befürchten. Auch die Gefahr einer gewebetoxischen Reaktion wurde vermutet.

Die Lösung der Aufgabe lag daher schon deshalb nicht nahe, weil es, wie bereits erläutert, durchaus problematisch ist, eine Kombination des Wirkstoffkomplexes mit einem Träger -hier einer Endoprothese- vorzusehen, weil dann die Funktionen des Wirkstoffkomplexes in dem Knochendefekt gestört sein oder zumindest durch mögliche Immunreaktionen kompliziert werden könnten.

Die zu stabilisierende Endoprothese weist eine äußere Fläche auf, die zumindest teilweise mit dem Wirkstoffkomplex beschichtet ist und/oder sie weist zumindest einen Hohlraum auf der mit dem Wirkstoffkomplex befüllt ist.

Durch das Beschichten und/oder Befüllen mit dem Wirkstoffkomplex soll ein schnelleres und dauerhaftes Einwachsen der Endoprothese im Organismus ermöglicht werden. Durch ein beschleunigtes und gleichzeitig verbessertes Einwachsen der Endoprothese an der implantierten Stelle resultiert eine längere Haltbarkeit und eine größere sowie zeitliche frühere Belastbarkeit der Endoprothese.

Gemäß einer weiteren Ausführungsform weist die Endoprothese zumindest einen Hohlraum auf, der mit dem Wirkstoffkomplex befüllt ist, wobei der Wirkstoffkomplex zusätzlich auf einem weiteren Trägermaterial aufgebracht ist. Als ein solches weiteres Trägermaterial kann Collagen oder ein geeignetes Polymer verwendet werden. Hierbei sind insbesondere die Collagene vom Typ I, IV, V, VII zu nennen. Die Collagene können beispielsweise in Form von Vliesen oder Gelen eingesetzt werden und weisen insbesondere eine an sich gute immunologische Verträglichkeit in Verbindung mit einer problemlosen Verarbeitung auf.

Bei den polymeren Trägermaterialien kommen insbesondere Polymere aus natürlichen Monomeren, wie Polyaminosäuren (Polylysin, Polyglutaminsäure, etc.) und Polymere der Milchsäure in Betracht. Es können auch Copolymere, z. B. aus Polymilchsäure und Hydroxyessigsäure, verwendet werden.
Polylaktate sind Polyester der Milchsäure mit der chemischen Formel:

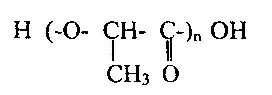

Bei der direkten Polymerisation der Monomere ergeben sich Polymere mit relativ niedrigen Molekulargewichten. Die obere Grenze liegt etwa bei 20000 Da. Höhere Molekulargewichte können durch die Verknüpfung zyklischer Dimere bei hoher Temperatur, geringem Druck und in Gegenwart von Katalysatoren entstehen. Die Milchsäurepolymere sind bioabbaubar, biokompatibel, wasserunlöslich und zeichnen sich durch eine große Festigkeit aus.

Die zusätzliche Verwendung eines weiteren Trägermaterials, wie Collagen oder die genannten Polymere, verringert die Menge des zum vollständigen Befüllen des Hohlraums der Endoprothese benötigten Wirkstoffkomplexes ohne seine grundsätzliche Wirksamkeit zu beeinträchtigen. Dadurch wird die Verwendung des Wirkstoffkomplexes wirtschaftlich günstiger.

Die Erfindung betrifft auch eine Endoprothese, nach Anspruch 4, die mit dem Wirkstoffkomplex in einer seiner verwendungsgemäßen Ausgestaltungen beschichtet ist oder die diesen aufweist.

Im folgenden wird die Erfindung anhand von Beispielen und Vergleichsbeispielen und unter Bezugnahme auf die beigefügte Zeichnung näher erläutert.

Es zeigen:
- Fig. 1:: eine schematische Darstellung der Knochenneubildung bei Kaninchen unter Verwendung des Wirkstoffkomplexes, im Vergleich zu einer Leerprobe,
- Fig. 2:: eine schematische Darstellung der Knochenneubildung bei Schafen unter Verwendung des Wirkstoffkomplexes, mit Tricalciumphosphat als Trägermaterial, im Vergleich zu reinem Tricalciumphosphat,
- Fig. 3:: eine schematische Darstellung der Knochenneubildung bei Ratten unter Verwendung des Wirkstoffkomplexes, mit verschiedenen Collagenen als Trägermaterial, im Vergleich zu den reinen Collagenen,
- Fig. 4a:: eine Seitenansicht einer für die Beschichtung mit dem Wirkstoffkomplex verwendeten Endoprothese
und
- Fig. 4b:: eine weitere Seitenansicht der Endoprothese, die gegenüber Fig. 4a um 90° C gedreht ist.

### I. Herstellung des Wirkstoffkomplexes

Im folgenden werden die wesentlichen Herstellungsschritte des Wirkstoffkomplexes beschrieben:
Röhrenknochen von Kälbern, Schafen, Kaninchen oder Ratten wurden gesäubert und unter anderem vom Knochenmark befreit und dann eingefroren. Der gefrorene Knochen wird auf eine Partikelgröße von weniger als 2 mm zerkleinert. Die zerkleinerten Knochenstücke wurden in Aceton entfettet und in 0.6 N Salzsäure entkalkt. Danach wird lyophilisiert und man erhält eine demineralisierte Knochenmatrix, die in 4 molarer Guanidinium-HCl-Lösung extrahiert wird. Die Extraktionslösung wird gegen Aqua dest. dialysiert und der Wirkstoffkomplex durch Abzentrifugieren und Lyophilisieren im Präzipitat erhalten.

Diese grundsätzliche Herstellungsweise ist im nachfolgenden Flußdiagramm nochmals dargestellt.

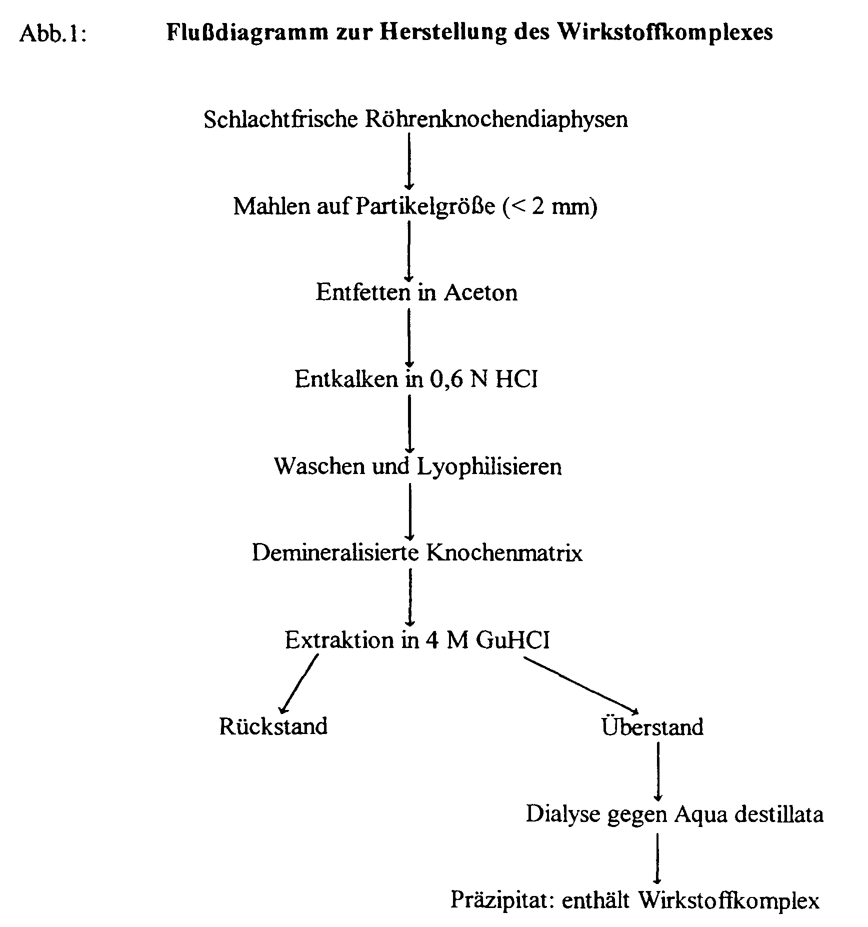

### II. Wirksamkeit des Wirkstoffkomplexes ohne Verwendung von Trägermaterialien

Um zu zeigen, daß der Wirkstoffkomplex als solches wirksam ist, wird zunächst ein Versuch dargestellt, bei dem der Wirkstoffkomplex ohne weitere Trägermaterialien implantiert wird.

### 1. Für den Versuch verwendete Tiere

Es werden weibliche Kaninchen der Rasse Chinchilla mit einem mittleren Körpergewicht von 3089 g verwendet. Sie erhielten Haltungsfutter für Kaninchen und mit Salzsäure auf pH 4,5 angesäuertes doppelt ozoniertes Leitungswasser nach Bedarf.

Die Tiere wurden durch Subcutaninjektion eines Gemisches von Ketamin und Xylazin narkotisiert.

### 2. Vorbereitung eines Knochendefektes bei den Kaninchen

Mit einem innen gekühlten Bohrer wurde ein Implantatlager von 4 mm Durchmesser und cirka 9 mm Tiefe im Kniegelenk (distales Femurende) des Kaninchens präpariert. Dann wurde das so gebildete Bohrloch jeweils mit 30 und 90 mg des Wirkstoffkomplexes gefüllt, der hergestellt worden war, wie unter I. beschrieben. Jeweils ein weiteres Bohrloch diente in Form eines "Leerloches" zur Kontrolle der Knochenneubildung.

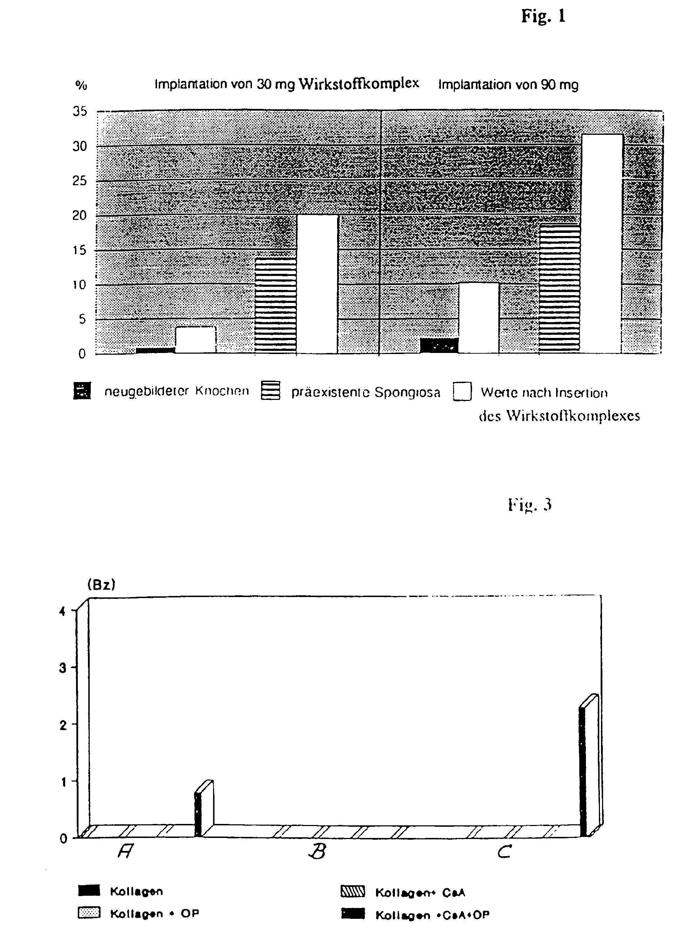

Fig. 1 zeigt die Knochenneubildung im Leerloch und im Bohrloch nach Implantation des Wirkstoffkomplexes sowie die Dichte der umgebenden präexistenten Spongiosa jeweils 28 Tage nach der Operation (n=2/Wirkstoff-Menge).

Bei der Auswertung der Versuche wurde festgestellt, daß die Dichte der die Bohrlöcher umgebenden Spongiosa nach Implantation von 30 mg des Wirkstoffkomplexes um 45 % und nach Implantation von 90 mg des Wirkstoffkomplexes um 69 % höher lag als beim Leerloch. Dabei hatte die Menge an präexistenter Spongiosa keinerlei Einfluß auf die Regeneration im Defekt, da die Knochenneubildung nach der Insertion des Wirkstoffkomplexes nicht von der Bohrlochperipherie ausging, sondern gleichmäßig über den Defekt verteilt war.

### III. Knochenbildung im Unterkiefer von Schafen unter Verwendung von Tricalciumphosphat (TCP)

Tricalciumphosphat (TCP) ist eine Calciumphosphatkeramik basierend auf dem System CaO/P₂O₅ und wird durch Pressen und anschließendes Sintern der Ausgangsmaterialien Calciumoxid (CaO) und Diphosphorpentoxid (P₂O₅) hergestellt. Alternativ kann es auch in einem Arbeitsschritt durch Heißpressen hergestellt werden.

### 1. Für die Versuche verwendete Tiere

Für die nachfolgend beschriebenen Untersuchungen wurden ausgewachsene Hausschafe von der Viehzentrale Südwest AG Stuttgart verwendet. Diese erhielten Heu und Wasser als Ernährung sowie drei Tage vor dem operativen Eingriff einen Brei aus Altromin-Pellets.

Die Tiere wurden mit 1 ml Xylazin/1ml Ketanest i. m. prämediziert. Dann wurden die Schafe mit Nembutal narkotisiert.

### 2. Vorbereitung des Implantats

TCP wurde in einer Lösung von 100 mg gelöstem Wirkstoffkomplex mit 10 ml Wasser suspendiert und unter ständigem Rühren mit flüssigem Stickstoff tiefgefroren. Nach 24-stündigem Lyophilisieren und anschließender Gassterilisation (Ethylenoxid) wurde das so mit dem Wirkstoffkomplex dotierte TCP in den nachfolgend beschriebenen Unterkiefer-Defekt eines Schafes eingebracht. Außerdem wurde ein weiterer Unterkiefer-Defekt, der als Vergleich diente, mit undotiertem, im Autoclaven sterilisiertem TCP gefüllt.

### 3. Vorbereitung des Unterkiefer-Defektes beim Schaf

In einem entsprechend vorbereiteten Unterkiefer eines Schafes wurde unter Kühlung mit physiologischer Kochsalzlösung mittels eines Trepanbohrers von 5 mm Durchmesser jeweils ein normierter Knochenzylinder herausgefräßt und entfernt. Dann wurde eines der so gebildeten Bohrlöcher mit TCP, das gemäß der Versuchsvorschrift 1. mit dem Wirkstoffkomplex dotiert worden war, befüllt und das zweite Bohrloch mit undotiertem TCP aufgefüllt.

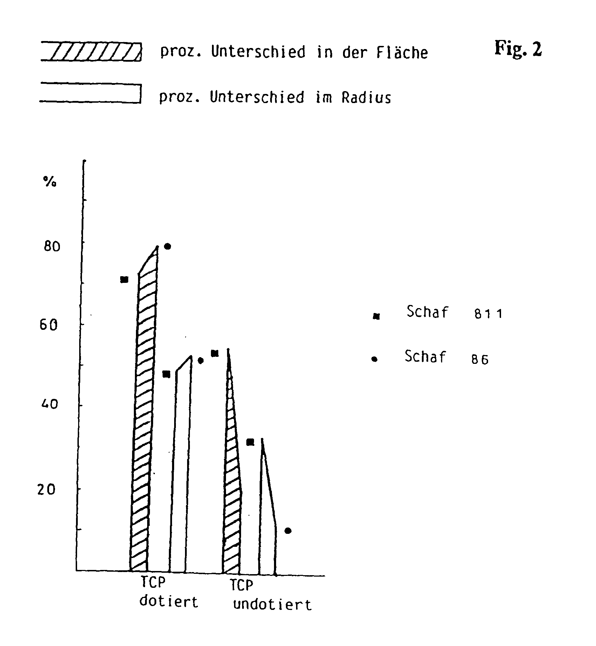

Die Ergebnisse des Knochenwachstums in den Unterkieferdefekten sind in Fig. 2 zur Erleichterung der Übersicht grafisch dargestellt. Die Versuchsdauer betrug 26 bzw. 41 Tage.

Es zeigte sich, daß durch die Dotierung von TCP mit dem Wirkstoffkomplex eine Beschleunigung der knöchernen Regeneration des Unterkieferdefektes der beiden Schafe Nr. 811 und 86 in der Anfangsphase von etwa 100 % erreicht wurde. Nach 41 Tagen betrug die Steigerung der Beschleunigung der knöchernen Regeneration immer noch 10 %. Die Knochenheilung verläuft daher besonders am Beginn deutlich schneller als ohne die osteoproduktive Wirkung der mit dem Wirkstoffkomplex dotierten Implantate.

Diese Erkenntnis ist insbesondere für die Beschichtung von Endoprothesen mit dem Wirkstoffkomplex von Bedeutung. Eine mit dem Wirkstoffkomplex beschichtete Endoprothese, beispielsweise im Fall eines Oberschenkelhals-Bruches, ermöglicht entsprechend ein schnelleres Einwachsen der Prothese und damit eine schnellere Regeneration und Rekonvaleszenz des jeweiligen Patienten. Dadurch wird die Dauer des Krankenhausaufenthaltes verkürzt.

### IV. Versuche mit Collagenen als Trägermaterialien

Der bereits bekannte, weiter oben erläuterte Wirkstoffkomplex kann zur Einheilung von Endoprothesen verwendet werden. Bei der Herstellung des Wirkstoffkomplexes ist der mengenmäßige Ertrag in dem erforderlichen Reinheitsgrad sehr gering. Daher wurde untersucht, ob es Trägermaterialien gibt, die mit dem Wirkstoffkomplex kombiniert werden können, um so die Menge des für die jeweilige Zielsetzung benötigten Wirkstoffkomplexes zu reduzieren, ohne dadurch seine knochenbildende Wirksamkeit zu verringern.

### 1. Wirkstoffkomplex

Der für die Zwecke der nachfolgend beschriebenen Versuche verwendete Wirkstoffkomplex wurde genauso hergestellt wie unter I. beschrieben, wobei Röhrenknochen von Kälbern verwendet wurden.

### 2. Versuchstiere

Es wurden männliche Wistarratten eines Gewichtes zwischen 350 und 400 g verwendet und in einem klimatisierten Tierstall bei 23° C und etwa 50 % relativer Luftfeuchtigkeit gehalten. Ernährt wurden sie mit einer Haltungsdiät für Ratten und Mäuse.

Jedem untersuchtem Tier wurden zwei Implantate desselben Trägermaterials in die Bauchmuskulatur eingebracht, von denen eins mit dem Wirkstoffkomplex beschichtet war, während das andere als Vergleichsimplantat unbeschichtet blieb. Die Tiere wurden nach 21 Tagen getötet und die betreffenden Areale der Implantate in der Bauchmuskulatur explantiert und histologisch ausgewertet.

### 3. Verwendete Trägermaterialien

Für diese Versuche wurden Collagenmaterialien eingesetzt, die alle käuflich zu erwerben sind. Collagen A war ein reines, steriles, natives, resorbierbares Rinderhautcollagen, das frei ist von jeglichen Fremdzusätzen, wie Stabilisatoren oder Desinfizienzien.
Collagen B war ein gereinigtes, lyophilisiertes, leicht quervernetztes steriles und nicht pyrogenes Rinderhautcollagen mit schwachen antigenen Eigenschaften. Die helikale Struktur des Collagens blieb erhalten.
Collagen C bestand aus reinen, nativen und resorbierbaren Rindercollagenfibrillen.

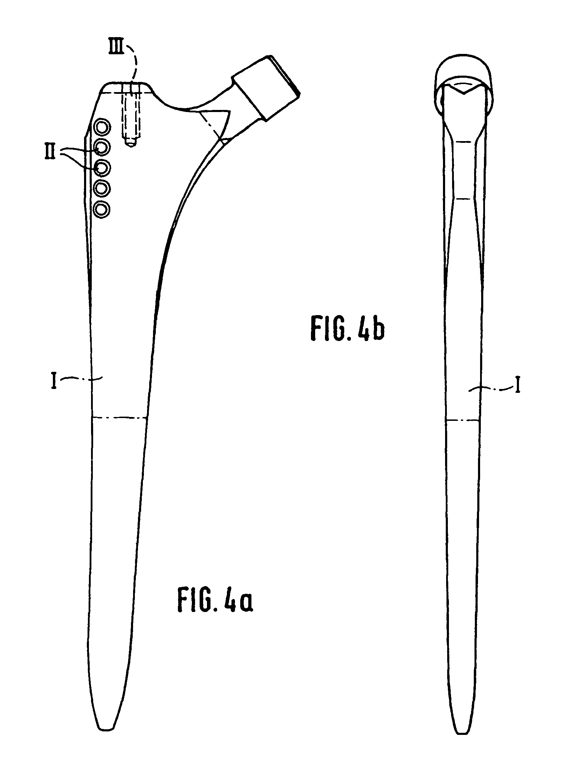

Alle verwendeten Collagene lagen in Vliesform vor. Es wurden Collagenvliesstücke von je 50 mg abgeschnitten und je 1 ml der Wirkstoffkomplex-Lösung (3mg/ml) zugegeben. Bei den Kontrollimplantaten wurde stattdessen 1 ml destillierten Wasser zugegeben. Die so behandelten Collagenvliesstücke wurden bei - 20° C eingefroren, lyophilisiert und ergaben Implantate mit einem Durchmesser von etwa 10 mm und einer Dicke von etwa 5 mm. Fig. 3 zeigt die Ergebnisse der Knochenbildung der Collagenimplantate A, B und C mit und ohne Beschichtung mit dem Wirkstoffkomplex sowohl in mit Cyclosporin A immunsupprimierten Tieren als auch in nicht immunsupprimierten Tieren nach 21 Tagen. Dabei entspricht die Bewertungszahl (BZ) dem arithmetischen Mittel der Bewertungszahlen von drei unabhängigen Personen bei sechs Implantaten jeder Gruppe.

Mit dem Wirkstoffkomplex beschichtetes Collagen A zeigte nach dieser Zeit bei immunsupprimierten Tieren einen knochenbildenden Effekt, während dieser bei Collagen B nicht nachgewiesen werden konnte. Demgegenüber zeigte jedoch Collagen C einen sehr ausgeprägten knochenbildenden Effekt.

Daraus folgt, daß es auf die Präparation des jeweils verwendeten Collagens ankommt und sich seine Eignung als Trägermaterial daraus ergibt. Collagene, die immunogen sind, sind zur Verwendung als Trägermaterialien ungeeignet.

### IV. Prüfung von Trägermaterialien auf ihre Biokompatibilität

In Versuchen, die die Verbesserung der Langzeitstabilität von Endoprothesen betrafen, wurden Titanplättchen mit unterschiedlicher Rauhigkeit (100, 20 und 0,5 µm), eine TiAl₆V₄-Legierung (0,5µm) und Al₂O₃Plättchen der Firma Friedrichsfeld sowie Hydroxylapatit-Plättchen von der Feldmühle AG eingesetzt. Hydroxylapatit wird durch keramisches Brennen von Pentacalciumhydroxid-Triphosphatpulver bei 1250°C gewonnen. Außerdem kann zur Herstellung einer Hydroxylapatitkeramik auch ein natürliches Material herangezogen werden, wie das carbonatische Skelett der Rotalge. Dabei werden nach einem Wasch- und Trocknungsvorgang zunächst die organischen Bestandteile durch Pyrolyse bei einer Temperatur von etwa 700°C entfernt. Dann erfolgt die Umsetzung zu Hydroxylapatit unter Hinzufügung von Phosphatlösung bei erhöhtem Druck und erhöhter Temperatur.

Bei einem weiteren Herstellungsverfahren einer Hydroxylapatitkeramik, ausgehend von dem natürlichen Skelett von Korallen, wird das Calciumcarbonat der Korallen durch hydrotermale Umwandlung in Hydroxylapatit oder eine Mischung aus Hydroxylapatit und weiteren mineralischen Strukturen umgewandelt. Bei dem so entstehenden Material bleibt die koralline Struktur, d. h. insbesondere das interkonnektierende Porensystem der Koralle, erhalten.

Die Beschichtungen mit dem Wirkstoffkomplex, welcher nach dem weiter oben angegebenen allgemeinen Verfahrensschema hergestellt worden war, wurden durch das Beschichtungsverfahren "Dip-coating" aufgebracht. Unter "Dip-coating" wird ein Beschichtungsverfahren verstanden, bei dem der zu beschichtende Gegenstand, hier die Plättchen, in eine Lösung mit einer gewünschten, vorgegebenen Konzentration des Beschichtungsmittels, hier des Wirkstoffkomplexes, getaucht wird. Anschließend wird lyophilisiert. Es werden dünne Überzugsschichten bzw. Beschichtungen erhalten. Die Prüfung der angegebenen Materialien auf ihre Biokompatibilität wurde insbesondere in Bezug auf die Rauhigkeit der Oberflächen durchgeführt (n=20; je vier Plättchen).

Bei dieser Biokompaktibilitätsprüfung der untersuchten Materialien zeigte sich, daß Titan aufgrund der höchsten Anzahl lebender Zellen sowie den besten Verhältnis lebender : toter Zellen als Trägermaterial sehr geeignet ist. Während Hydroxylapatit ein ähnlich gutes Ergebnis lieferte schnitt TiAl₆V₄ erheblich schlechter ab.

Generell zeigte sich in Bezug auf die Oberflächenrauhigkeiten, daß die glattesten Oberflächen, d. h. Oberflächen mit einem Porendurchmesser von 0,2 - 0,5 µm, mit Ausnahme von TiAl₆V₄ die besten Resultate lieferten. Mit Zunahme der Rauhigkeit bzw. des Porendurchmessers sinken sowohl die Anzahl lebender Zellen als auch das Verhältnis lebender : toter Zellen. Bei einem Porendurchmesser von etwa 0,5 µm wurde der höchste Anteil an lebendem (Knochen)-Gewebe in unmittelbarem Kontakt zur Plättchengrenzfläche erhalten.

**Tabelle 1:**

| Trägermaterial | | Anzahl lebender Zellen/cm² | Anzahl toter Zellen/cm² |
|---|---|---|---|
| Hydroxylapatit | | | |
| | 0,2 - 0,5 µm | 1792 ± 700 | 200 ± 37 |
| | 20 µm | 7469 ± 2614 | 2238 ± 715 |
| | 50 µm | 4477 ± 408 | 1692 ± 427 |
| Osprovit (Feldmühle) | | 7930 ± 2007 | 1638 ± 377 |
| Titan | 0,5 µm | 11377 ± 2538 | 1054 ± 308 |
| | 20 µm | 9600 ± 3038 | 1754 ± 439 |
| | 100 um | 2308 ± 669 | 2085 ± 623 |
| TiAl₆V₄ | 0,5 µm | 7200 ± 1062 | 2800 ± 954 |
| Al₂O₃ | reinst, poliert | 11446 ±1500 | 2292 ± 600 |

Die Ergebnisse dieser Versuche konnten nun auf die Beschichtung von Endoprothesen mit dem Wirkstoffkomplex übertragen werden. Eine Draufsicht auf die dabei verwendete Endoprothese ist in Fig. 4 dargestellt.

Vor dem Einsetzen der Endoprothesen wurden deren äußere Oberfläche (I) nach dem Verfahren des "Dip-coatings" mit dem Wirkstoffkomplex beschichtet und zusätzlich wurde der Wirkstoffkomplex in die inneren Hohlräume des Prothesenschaftes (II), die Austritte an die Schaftoberfläche haben, eingeführt. Dadurch ergibt sich für zukünftige mögliche Lockerungen der Endoprothesen der Vorteil, daß nachträglich der Wirkstoffkomplex ohne einen größeren Eingriff aufgebracht werden kann und dort zu einer Knochenbildung und damit Verfestigung der Endoprothese führt. Wahlweise kann auch im Bereich der Schraubverbindung **(III)** eine Beschichtung mit dem Wirkstoffkomplex vorgesehen sein.

Daß die Beschichtung mit dem Wirkstoffkomplex zu höheren Belastungsmöglichkeiten im Vergleich zu nicht beschichteten Oberflächen führt, zeigt Tabelle 2 am Beispiel von Hydroxylapatit (HA). Dabei wurden die Zugfestigkeiten an der Grenzfläche verschiedener Implantatmaterialien in N/mm² ± Standardabweichung bestimmt. Als Material wurde mittels heiß isostatischem Pressen (HIP) hergestellter Hydroxylapatit gegenüber zusätzlich mit dem Wirkstoffkomplex beschichtetem Hydroxylapatit bestimmt. Das Implantatmaterial wurde in das distale Kaninchenfemur implantiert und nach 84 Tagen untersucht. Die dabei aufgefundenen Zugfestigkeiten sind in der nachfolgenden Tabelle angegeben.

**Tabelle 2**

| Material | RT (µm) | Tage | n | Zugfestigkeit |
|---|---|---|---|---|
| HA HIP | 0,5 | 84 | 10 | 1,53 ± 0,24 |
| HA HIP WK | 0,5 | 84 | 6 | 2,27 ± 0,31 |

| | | | | |
|---|---|---|---|---|
| n= Anzahl der Implantate WK= Beschichtung mit dem Wirkstoffkomplex HIP= heiß isostatisches Pressen RT= Oberflächenrauhigkeit | | | | |

Es wird abschließend noch darauf hingewiesen, daß es sich bei den für die Zwekke der vorliegenden Erfindungen dargestellten Versuchen immer dann um sorgfältig ausgesuchte Modelluntersuchungen handelt, wenn der eigentliche Gegenstand, die z. B. im Bereich des Oberschenkelhalsknochens implantierte Endoprothese, für die Untersuchungen nicht zur Verfügung stehen konnte, weil es sich hierbei um unzumutbare Versuche am menschlichen Körper gehandelt hätte.

Außerdem ist die Erfindung auf alle denkbaren Endoprothesen anwendbar. Die Beschreibung am Beispiel der Endoprothese im Bereich des Oberschenkelhalses hat exemplarischen Charakter.

## Patentansprüche

1. Verwendung eines Wirkstoffkomplexes für die Herstellung von Knochen mit den folgenden, voneinander unterschiedlichen und auf die Herstellung von Knochen spezifisch abgestimmten Komponenten in Form mindestens einer Strukturkomponente, ausgewählt aus Collagen, Elastin oder Proteoglycanen, mindestens einer Rekrutierungskomponente, ausgewählt aus chemotaktischen Peptiden, Metaboliten der Arachidonsäure oder Mischungen davon, mindestens einer Adhäsionskomponente, ausgewählt aus Fibronectin, Laminin, L-CAM, N-CAM, Cytotactin, Tenascin, Collagenen vom Typ IV, V, VII, synthetischen Peptiden und Transmembran-Verbindungsproteinen, und mindestens einer Wachstums- und/oder Maturationskomponente, ausgewählt aus einem oder mehreren Cytokinen, zur Herstellung eines Endoprothesen-Implantats, wobei die Endoprothese eine äußere Fläche (I) aufweist, die zumindest teilweise mit dem Wirkstoffkomplex beschichtet ist und/oder zumindest einen Hohlraum (II) aufweist, der mit dem Wirkstoffkomplex befüllt ist.

2. Verwendung nach Anspruch 1, **dadurch gekennzeichnet, daß** die Endoprothese zumindest einen Hohlraum (II) aufweist, der mit dem Wirkstoffkomplex befüllt ist, wobei der Wirkstoffkomplex zusätzlich auf einem weiteren Trägermaterial aufgebracht ist.

3. Verwendung nach Anspruch 2, **dadurch gekennzeichnet, daß** als weiteres Trägermaterial Collagen, mit Ausnahme von immunogenem Collagen, oder ein Polymer verwendet wird.

4. Endoprothese, die mit dem Wirkstoffkomplex nach einem der Ansprüche 1 bis 3 beschichtet oder befüllt ist.

## Claims

1. The use of an active substance complex for creating bone with the following components which differ from one another and are specifically adapted for creating bone, namely at least one structural component selected from the group consisting of collagen, elastin, or proteoglycans, at least one recruiting component selected from the group consisting of chemotactic peptides, metabolites of arachidonic acid, or mixtures thereof, at least one adhesion component selected from the group consisting of fibronectin, laminin, L-CAM, N-CAM, cytotactin, tenascin, collagen types IV, V, VII, synthetic peptides and transmembrane compound proteins, and at least one growth and/or maturation component, selected from the group consisting of one or more cytokins, for producing an endoprosthesis implant, whereby the endoprosthesis has an outer surface (I) which is coated at least partially with the active substance complex and/or has at least one cavity (II) which is filled with the active substance complex.

2. The use as claimed in claim 1, **characterized in that** the endoprosthesis has at least one cavity (II) which is filled with the active substance complex, the active substance complex being additionally applied to a further support material.

3. The use as claimed in claim 2, **characterized in that** collagen, with the exception of immunogenic collagen, or a polymer is used as further support material.

4. An endoprosthesis which is coated with the active substance complex as claimed in one of claims 1 through 3 or which is filled with said active substance complex.

## Revendications

1. Utilisation d'un complexe d'agents actifs pour la fabrication d'os avec les composants suivants, différents les uns des autres et ajustés spécifiquement pour la fabrication d'os, sous la forme d'au moins un composant de structure, choisi parmi des collagènes, l'élastine ou des protéoglycanes, d'au moins un composant de recrutement, choisi parmi des peptides chimiotactiques, des métabolites de l'acide arachidonique ou de mélanges de ceux-ci, d'au moins un composant d'adhésion, choisi parmi la fibronectine, la laminine, L-CAM, N-CAM, la cytotactine, la ténascine, des collagènes de type IV, V, VII, des peptides synthétiques et des protéines de liaison transmembranaires, et d'au moins un composant de croissance et/ou de maturation, choisi parmi une ou plusieurs cytokines, pour la fabrication d'un implant d'endoprothèse, l'endoprothèse présentant une surface extérieure (I) qui est au moins partiellement revêtue du complexe d'agent actifs et/ou présente au moins une cavité (II) remplie du complexe d'agents actifs.

2. Utilisation selon la revendication 1, **caractérisée en ce que** l'endoprothèse présente au moins une cavité (II) remplie du complexe d'agents actifs, le complexe d'agents actifs étant appliqué en plus sur un matériau support supplémentaire.

3. Utilisation selon la revendication 2, **caractérisée en ce qu'**on utilise comme matériau support supplémentaire du collagène, à l'exception des collagènes immunogènes, ou un polymère.

4. Endoprothèse revêtue ou remplie avec le complexe d'agents actifs selon l'une des revendications 1 à 3.
